# EUROPEAN PATENT APPLICATION

(11) **EP 4 644 225 A1**
(43) Date of publication of application: **05.11.2025**
(21) Application number: 25171935.7
(22) Date of filing: 23.04.2025
(51) Int. Cl.: B63B 22/04, C02F 3/20, G01N 33/18, B63B 22/18, B66C 23/02, G01K 13/02, G01N 27/28, G01N 27/409, G01N 27/416, G08C 17/02

(54) **FLOATING WATER QUALITY MEASURING DEVICE**

(30) Priority: 30.04.2024 KR 20240058053
(71) Applicant: Ecosense Co., Ltd., Seoul 08501 (KR)
(72) Inventor: KWON, Dong Myung, 08501 Seoul (KR); KOO, Ja Hoon, 11912 Gyeonggi-do (KR); CHOI, Jae Woong, 14651 Gyeonggi-do (KR)
(74) Representative: Byrne, Declan

(57) **Abstract**

The present invention provides a floating water quality measuring device (1) for measuring the quality of water treated in a sewage treatment plant. The floating water quality measuring device (1) includes a cantilever unit (10), a wire rope (20), and a floating sensor unit (30).

## Description

### [CROSS REFERENCE TO RELATED APPLICATIONS]

This application claims the benefit of Korean Patent Application No. 10-2024-0058053 filed on April 30, 2024, the entirety of which is incorporated by reference herein.

### [BACKGROUND OF THE INVENTION]

### 1. Field of the Invention

The present invention relates to water quality measurement technology, and more particularly, to a device for measuring the quality of water treated in a sewage treatment plant.

### 2. Description of the Related Art

With the improvement of living standards and the development of industry, the amount of water used and wastewater discharged have been increasing. Accordingly, the volume of sewage treated in sewage treatment plants is also increasing.

Currently, most sewage treatment plants are equipped with facilities such as sedimentation tanks and biological reactors to purify sewage. When sewage is purified through such facilities, the purification is carried out through several stages, including flow rate adjustment in a large amount of sewage, pH adjustment, reduction, neutralization, reaction, coagulation, and final treatment.

The sewage treatment plant monitors the purification status of each stage and controls the purification process by adjusting conditions of each stage based on the extracted data.

A water quality measuring device installed in the reaction tank measures parameters such as dissolved oxygen and the overall quality of the treated water. It also monitors the condition of the treated water and extracts condition data from the treated water filled in the reaction tank.

However, conventional water quality measuring devices are susceptible to overturning due to water currents generated by water level fluctuations in the sewage treatment plant, which may hinder accurate water quality measurement.

In addition, when the sensor is exposed to air due to overturning, there is a concern that the sensor may fail more quickly. Furthermore, since the conventional water quality measuring devices are fixed to a railing installed in the reaction tank, it is difficult for a manager to perform maintenance. In addition, when foreign substances in the sewage adhere to the measuring device, it is difficult to remove them easily.

### [Prior Art Document]

### [Patent Document]

(Patent Document 1) Korean Patent Registration No. 10-1503688 entitled 'Floating Wireless Off-Gas Measuring Device'

### (registered on March 12, 2015)

### [SUMMARY OF THE INVENTION]

An object of the present invention is to provide a floating water quality measuring device that addresses problems such as the inability to move flexibly in response to changes in water level and water currents in a sewage treatment plant, the risk of overturning and resulting accelerated sensor failure, and difficulty in maintenance due to foreign substances attaching and adhering to an upper surface of the device.

The problems to be solved by the present invention are not limited to those mentioned above, and other technical problems not explicitly stated may be clearly understood by those skilled in the art based on the following descriptions.

To achieve the above-described object, there is provided a floating water quality measuring device including: a cantilever unit including a support module, a column-axis module, and a fixed plate module, wherein the support module includes a first wire fixing ring formed on a lower surface thereof, a pivot hole vertically penetrating from an upper surface to the lower surface at one end, and a column through-hole vertically penetrating from the upper surface to the lower surface at a position spaced apart from the pivot hole toward the other end, wherein a lower end of the column-axis module passes through the column through-hole of the support module and protrudes outward from the support module, and wherein the fixed plate module includes a support end which supports the lower end of the column-axis module protruding outward from the support module, and a fixing pin insertion hole formed at a position overlapping with the pivot hole; a wire rope having one end connected to the first wire fixing ring; and a floating sensor unit including a floating module configured to float on water, a cover module including a sensor connector formed on a lower surface thereof, which is connected at an upper surface thereof to the other end of the wire rope, thereby being fixed to the upper surface of the floating module, and a sensor module having one end connected to the sensor connector and the other end protruding outside the floating module to measure water quality.

The cover module may be formed in a hat shape inclined downward from top to bottom so that fluid flows from the upper side to the lower side.

The support module may include: a pulley formed on a lower surface thereof; and a wire detachment prevention member including: a housing formed at a position spaced apart from the pulley; a first wheel installed on one side of the housing; and a second wheel installed on the other side of the housing, wherein the wire rope may pass between the first wheel and the second wheel.

The column-axis module may include a column fixing ring formed at an upper end thereof, to which one end of the fixing wire is connected, and the support module may include a second wire fixing ring formed on an upper surface thereof, to which the other end of a fixing wire connected to the column fixing ring is connected.

The sensor module may include: a first sensor configured to measure dissolved oxygen (DO) of water; and second to Nth sensor configured to measure at least one of hydrogen ion concentration (pH), oxidation-reduction potential (ORR), temperature, and chemical oxygen demand (COD) of water, wherein the sensor module may be detachably coupled to the sensor connector.

The fixed plate module may include at least one lower fixing hole formed at a position spaced apart from the fixing pin insertion hole, into which a fixed bolt is inserted.

The floating water quality measuring device may further include a first weight connected to the wire rope located between the first wire fixing ring and the first pulley.

The floating module may be formed of a donut-shaped plastic tube.

The floating water quality measuring device according to the present invention may measure water quality while maintaining a stable posture and a constant position, even when the water level in the aeration tank fluctuates or when water currents occur. In addition, the present invention allows foreign substances to naturally flow off without adhering to the upper surface of a flowing unit, thereby preventing contamination of the upper surface of the flowing unit caused by such substances.

Furthermore, the present invention is configured such that one end of the cantilever unit is installed on the upper surface of the wall of the water quality measuring facility, and the cantilever unit is able to pivot both clockwise and counterclockwise between an initial installation position and a pivoted position, thereby allowing easier maintenance by the manager.

### [BRIEF DESCRIPTION OF THE DRAWINGS]

The above and other objects, features and other advantages of the present invention will be more clearly understood from the following detailed description taken in conjunction with the accompanying drawings, in which:
FIG. 1 is a schematic view illustrating a floating water quality measuring device according to an embodiment of the present invention;
FIGS. 2 and 3 are schematic views illustrating a cantilever unit of FIG. 1;
FIGS. 4 and 5 are schematic views illustrating a floating sensor unit of FIG. 1;
FIG. 6 is a schematic view illustrating first to Nth sensor modules connected to a sensor connector of FIG. 5;
FIG. 7 is a schematic view illustrating the operating state of the floating water quality measuring device according to an embodiment of the present invention;
FIGS. 8 and 9 are schematic views illustrating the state in which a support module of the cantilever unit of the present invention is pivoting; and
FIG. 10 is a perspective view illustrating the state in which a cover module of the floating sensor unit of the present invention is formed in a hat shape, thereby allowing water to flow downward.

### [DETAILED DESCRIPTION OF THE INVENTION]

The above and other aspects, features, and advantages of the present invention will become apparent from the detailed description of the embodiments set forth below in conjunction with the accompanying drawings. In this regard, it should be understood that the present invention is not limited to the embodiments described below.

The present invention may be embodied in various different forms, and the embodiments disclosed herein are provided to fully disclose the scope of the invention and to provide a thorough understanding of the invention to those skilled in the art to which the present invention pertains. The scope of the present invention is defined only by the appended claims. Furthermore, the same reference numerals used throughout this specification refer to the same components.

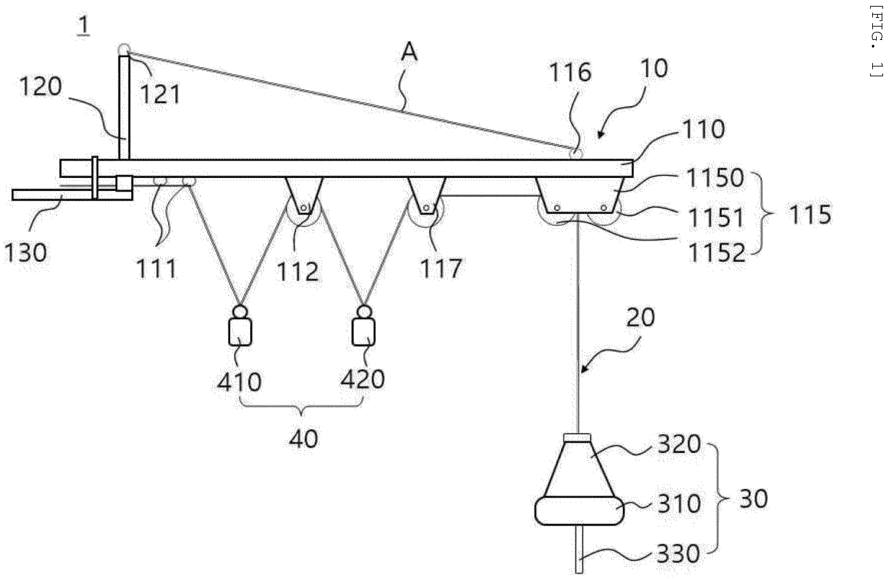

Hereinafter, in order to make the description of the present invention concise and clear, a floating water quality measuring device of the present invention will be generally described with reference to FIG. 1. Hereinafter, the components constituting the device of the present invention will be described in detail with the accompanying drawings.

A floating water quality measuring device 1 according to the present invention may maintain a stable posture and a constant position even when the amount of treated water in a water quality enhancement tank (aeration tank) fluctuates or when strong water currents of the treated water occur. Therefore, water quality can always be measured stably. Furthermore, the floating water quality measuring device 1 according to the present invention includes a cantilever unit 10 having one end fixed and the other end capable of pivoting clockwise and counterclockwise about the one end between an initial installation position and a pivoted position. Accordingly, the manager may easily maintain the water quality measuring device by pivoting the cantilever unit 10 of the present invention at a predetermined angle from the initial installation position to the pivoted position, thereby making it easier to maintain the water quality measuring device than with conventional water quality measuring devices.

Furthermore, the floating water quality measuring device 1 according to the present invention includes a floating sensor unit 30 having a cover module formed in a hat shape. Therefore, even if water rises to the cover module of the floating sensor unit as the water flow surges, the water may be drained downward. Therefore, by preventing water stains and foreign substances from accumulating on the upper surface of the floating sensor unit, contamination of the floating sensor unit may be prevented.

The floating water quality measuring device 1 configured as described above includes the cantilever unit 10, a wire rope 20, and the floating sensor unit 30 as components.

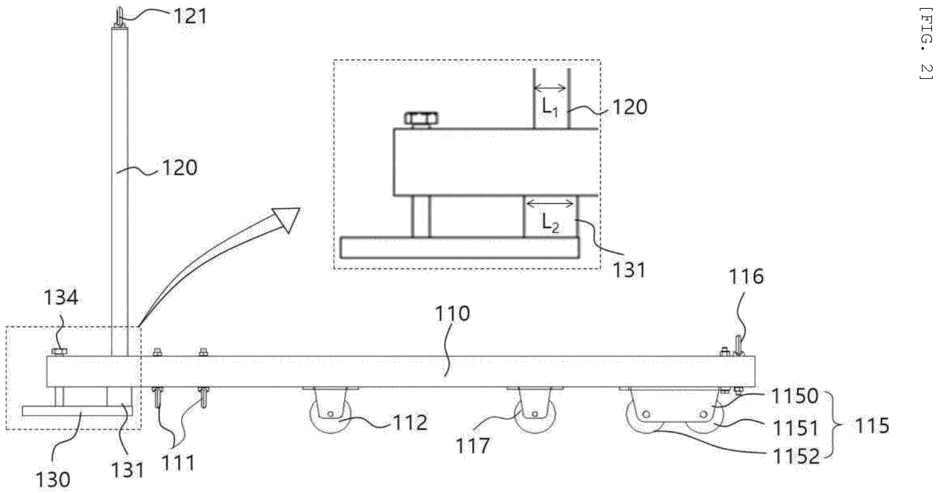

Hereinafter, the components of the floating water quality measuring device will be described in detail with reference to FIGS. 2 to 6.

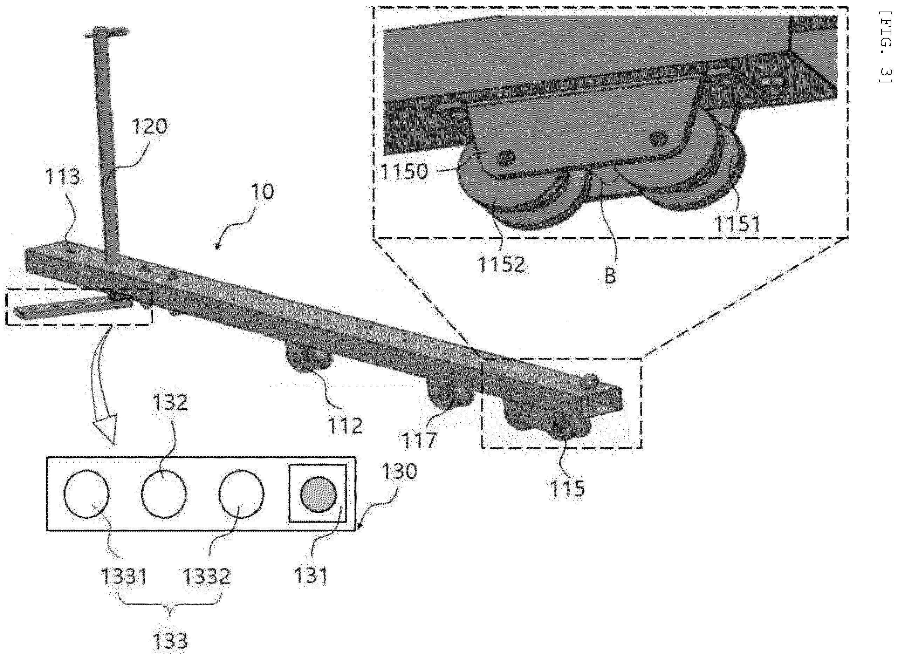

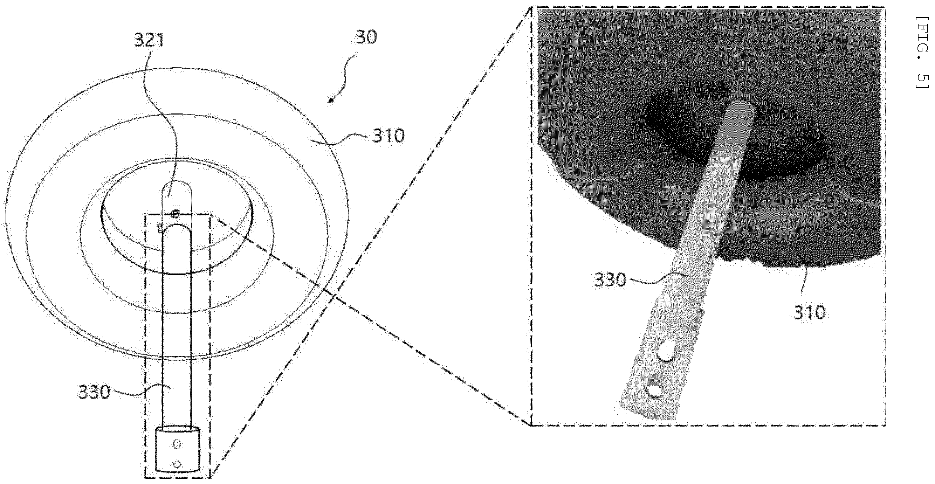

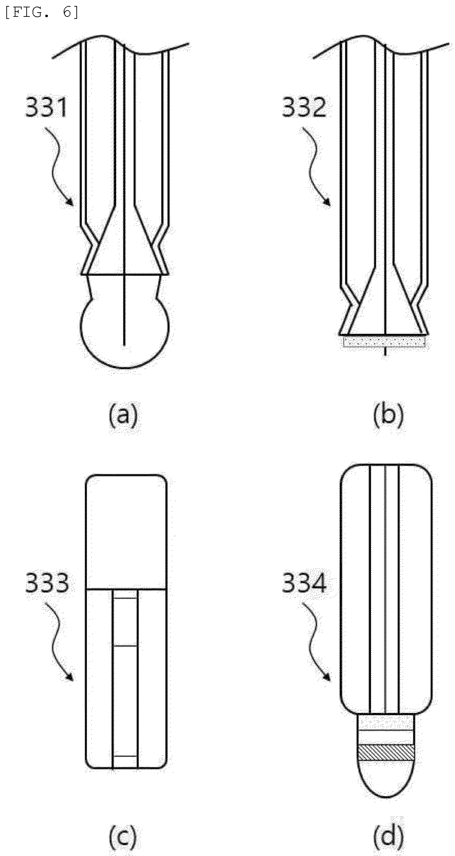

FIGS. 2 and 3 are schematic views illustrating the cantilever unit of FIG. 1, and FIGS. 4 and 5 are schematic views illustrating the floating sensor unit of FIG. 1. In addition, FIG. 6 is a schematic view illustrating first to Nth sensor modules connected to a sensor connector of FIG. 5.

The cantilever unit 10 includes one end installed on a wall of a water quality enhancement tank (aeration tank) C, and the other end extending from the one end perpendicular to the wall, thus to support the wire rope 20 and the floating sensor unit 30, etc. The other end of the cantilever unit 10 extends vertically from the wall of the water quality enhancement tank (aeration tank) so that the floating sensor unit 30 can be positioned at a specific position in the water quality enhancement tank (aeration tank) C by the wire rope 20. In addition, the cantilever unit 10 prevents the wire rope 20 from becoming detached from the cantilever unit 10 and the floating sensor unit 30 from becoming detached from the wire rope 20 even if the floating sensor unit 30 is shaken by the water current. In this case, the water current is generated by oxygen aeration, etc. rising from the bottom of the water quality enhancement tank (aeration tank) C. As shown in FIG. 2, the cantilever unit 10 includes a support module 110, a column-axis module 120, and a fixed plate module 130. Here, the support module 110 is formed as a rectangular hollow tube, i.e., a square tube. In addition, the support module 110 includes a first wire fixing ring 111 and a first pulley 112 formed on a lower surface thereof. In addition, the support module 110 includes a pivot hole 113 vertically penetrating from an upper surface to a lower surface at one end i.e., on the left side of the drawing, and a column through-hole 114 also vertically penetrating from the upper surface to the lower surface at a position spaced apart from the pivot hole 113 toward the other end. In addition, the support module 110 may include a wire detachment prevention member 115 formed on the lower surface thereof at a position spaced apart from the first pulley 112, and a second wire fixing ring 116 formed on the upper surface thereof. In this case, the wire detachment prevention member 115 includes a housing 1150 coupled to the other end of the support module 110, a first wheel 1151 installed on one side of the housing 1150, and a second wheel 1152 installed on the other side of the housing, spaced apart from the first wheel 1151. A gap B is formed between the first wheel 1151 and the second wheel 1152, through which the wire rope passes. One end of the wire rope 20 may pass through the gap B and extend downward to be connected to the floating sensor unit 30.

The wire detachment prevention member 115 prevents the wire rope 20 from becoming detached from the pulleys 112 and 117 due to water currents generated in the water quality enhancement tank (aeration tank) during the lowering process of the wire rope 20 via the gap B, through which the wire rope passes. In addition, the second wire fixing ring 116 is connected to the column-axis module 120 by a fixing wire A to prevent the support module 110 from tipping forward.

The column-axis module 120 has a lower end protruding outward from the support module 110 through the column through-hole 114 of the support module 110, and is installed horizontally along the wall of the water quality enhancement tank (aeration tank). In this case, the lower end of the column-axis module 120 is connected to the fixed plate module 130 which is directly fixed to the wall of the water quality enhancement tank (aeration tank). The fixed plate module 130 will be described after the column-axis module 120.

The support module 110 is installed vertically on the column-axis module 120 disposed horizontally along the wall of the water quality enhancement tank (aeration tank) C. This column-axis module 120 functions as a pivot axis, and the support module 110 may pivot clockwise and counterclockwise around the column-axis module 120. Furthermore, the column-axis module 120 includes a column fixing ring 121 formed at the upper end thereof. Accordingly, the other end of the support module 110 is securely supported by the column-axis module 120 via the fixing wire A, both ends of which are connected to the column fixing ring 121 and the second wire fixing ring 116. Therefore, the support module 110 can be prevented from tipping forward.

The fixed plate module 130 is fixed to an upper surface of the wall of the water quality enhancement tank (aeration tank) and supports the support module 110 at a position spaced apart from the upper surface of the wall of the water quality enhancement tank (aeration tank) so that the support module 110 may pivot clockwise and counterclockwise with respect to the fixed plate module 130. The fixed plate module 130 is formed in a plate shape and includes a support end 131 for supporting the lower end of the column-axis module 120 at one end. The fixed plate module 130 includes a fixing pin insertion hole 132 formed at a position overlapping with the pivot hole 113 of the support module 110, while the lower end of the column module 120 is supported by the support end 131. Here, the fixing pin insertion hole 132 functions as a hole into which the lower end of the fixing pin 134 is inserted. When the lower end of the fixing pin 134 passes through the pivot hole 113 and is inserted into the fixing pin insertion hole 132, the support module 110 is prevented from pivoting clockwise or counterclockwise around the column-axis module 120 by the fixed plate module 130. The fixed plate module 130 further includes a lower fixing hole 133 formed at a position spaced apart from the fixing pin insertion hole 132. For example, as shown in FIG. 3, the fixed plate module 130 may include a first lower fixing hole 1331 formed on one side and a second lower fixing hole 1332 formed on the other side, both spaced apart from the fixing pin insertion hole 132. One end of a fixed bolt attached to the upper surface of the wall of the water quality enhancement tank (aeration tank) C is inserted into the first lower fixing hole 1331 and the second lower fixing hole 1332 sequentially. Accordingly, the fixed plate module 130 may be securely fixed to the wall of the water quality enhancement tank (aeration tank) C.

The wire rope 20 serves to connect the floating sensor unit 30 to the cantilever unit 10. One end of the wire rope 20 is connected to the first wire fixing ring 111, and the other end is connected to the floating sensor unit 30, so that the floating sensor unit 30 may be hung on the cantilever unit 10. In this case, while one end of the wire rope 20 is connected to the first wire fixing ring 111 of the support module 110, the other end passes through at least one pulley 112 installed on the lower surface of the support module 110, for example, the first pulley 112 and the second pulley 117 as shown in FIG. 3, then passes through the wire detachment prevention member 115, and then connected to the floating sensor unit 30. In this way, since the wire rope 20 extends via the first pulley 112 and the second pulley 117, when the floating sensor unit 30 ascends, the wire between the first wire fixing ring 111 and the first pulley 112 and between the first pulley 112 and the second pulley 117 slackens as it moves downward. On the other hand, when the floating sensor unit 30 descends, the wire between the first wire fixing ring 111 and the first pulley 112 and between the first pulley 112 and the second pulley 117 tightens as it moves upward.

To this end, the wire rope 20 may be provided with weights 40 including a first weight 410 and a second weight 420 connected thereto at an interval. For example, as shown in FIG. 1, the first weight 410 may be installed on a wire between the first wire fixing ring 111 and the first pulley 112, and the second weight 420 may be installed on a wire between the first pulley 112 and the second pulley 117, so as to generate a tensile force corresponding to the floating position of the floating sensor unit 30. Accordingly, the wire rope 20 varies the tensile force generated between the first wire fixing ring 111 and the first pulley 112 and between the first pulley 112 and the second pulley 117, in response to the rising and falling of the floating sensor unit. Meanwhile, the tensile force is maintained between the wire detachment prevention member 115 and the connection to the floating sensor unit 30. As a result, when the water level rises and the floating sensor unit floats, the wire rope 20 may be pulled downward by the weight of the first weight 410 and the second weight 420, thereby preventing it from becoming entangled with the floating sensor unit 30.

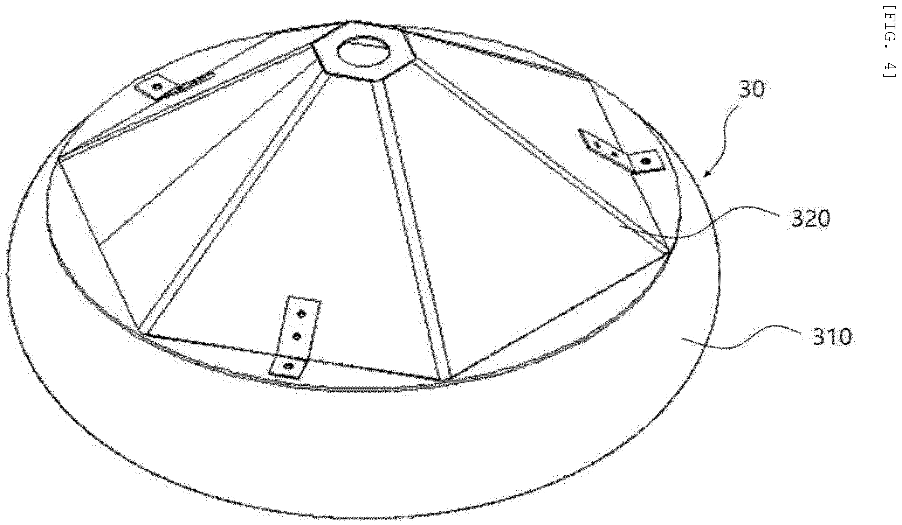

The floating sensor unit 30 is installed in a floating state in the water quality enhancement tank (aeration tank) C to measure the quality of water contained in the water quality enhancement tank (aeration tank) C. The floating sensor unit 30 includes a floating module 310, a cover module 320, and a sensor module 330. Here, the floating module 310 functions as a floating body configured to floats on water. For example, the floating module 310 may be formed of a donut-shaped plastic tube, as shown in FIG. 5. The cover module 320 is installed on an upper surface of the floating module 310. The cover module 320 functions as a cover to protect the upper surface of the floating module 310. The cover module 320 includes a sensor connector 321 formed on a lower surface thereof, and is connected at an upper surface thereof to one end of the wire rope 20, thereby being fixed to the upper surface of the floating module 310. As shown in FIG. 4, the cover module 320 may be formed in a hat shape.

The sensor module 330 may include, as shown in FIG. 6(a), a first sensor 331 configured to measure a first characteristic of water, i.e., dissolved oxygen (DO), a second sensor 332 configured to measure a second characteristic of water, i.e., hydrogen ion concentration (pH: potential of hydrogen), a third sensor 333 configured to measure a third characteristic of water, i.e., temperature, and an Nth sensor (33N) configured to measure oxidation-reduction potential (ORR) and chemical oxygen demand (COD). The first sensor 331 to the Nth sensor 33N may be detachably coupled to the sensor connector 321 formed on a lower surface of the cover module 320. In addition, the sensor module 330 may include a communication device capable of data communication, and may measure the characteristics of the water contained in the water quality enhancement tank (aeration tank) C, and then transmit the measured characteristics to a data transmission device D. Here, the data transmission device D transmits the received data to a control center that controls the operation of an aerator module (not shown) installed at the bottom of the water quality enhancement tank (aeration tank) . In this case, the control center may control the operation of a blower of the aerator module based on the data received from the data transmission device.

In this way, the data measured by the sensor module 330 is transmitted to the control center, and the operation of the aerator module of the water quality enhancement tank (aeration tank) is controlled based on the data received by the control center. Accordingly, the floating water quality measuring device 1 may function as a component of a system that monitors and manages water quality in real time.

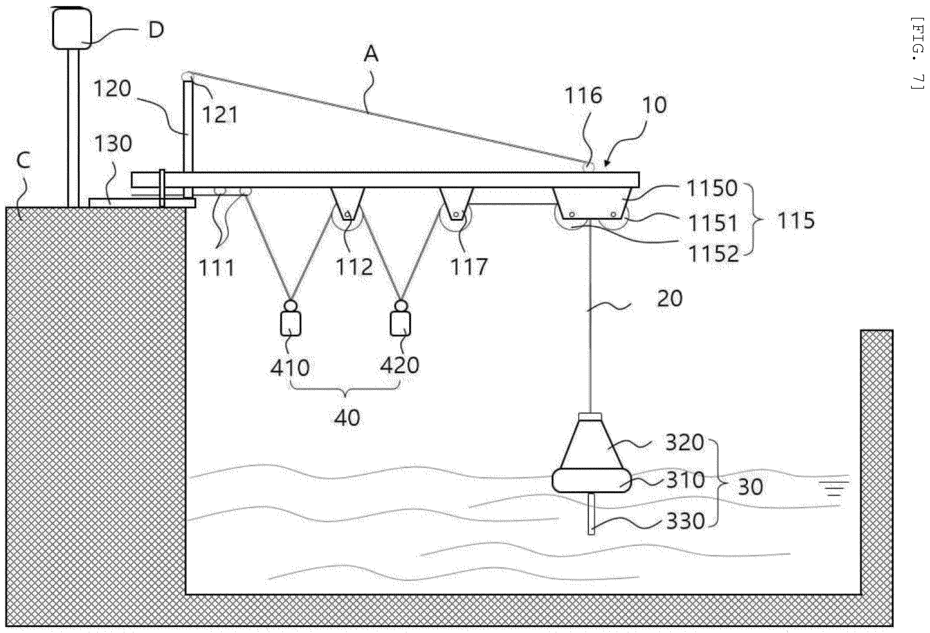

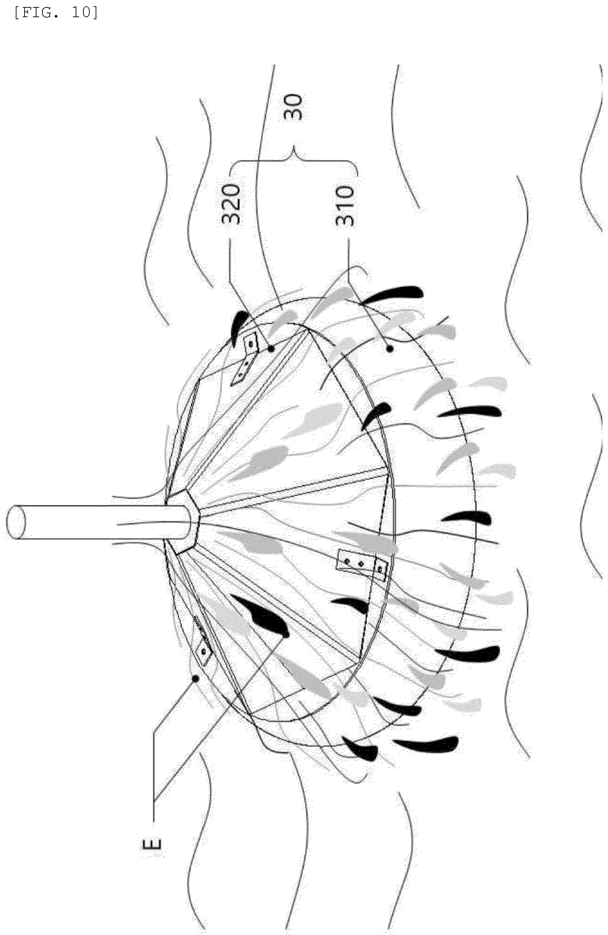

Hereinafter, the operation of the floating water quality measuring device 1 of the present invention will be described in detail with reference to FIGS. 7 to 10.

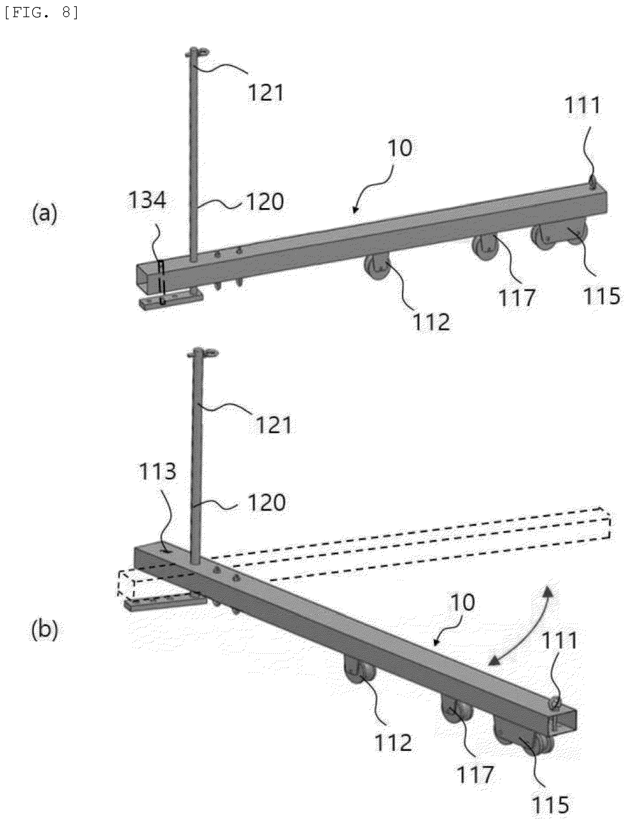

The floating water quality measuring device 1 of the present invention measures the characteristics of the water, i.e., the water quality, in the water quality enhancement tank (aeration tank) C, as shown in FIG. 7. The floating water quality measuring device 1 can maintain a constant water quality measuring position by automatically raising and lowering the floating sensor unit 30 even when water swells and currents are generated by an air diffuser installed in the water quality enhancement tank (aeration tank) C. In addition, even when the surging water contacts the cover module 320, the water is drained downward, thereby preventing accumulation of water in the cover module 320. Furthermore, as described above, the cantilever unit 10 of the floating water quality measuring device 1 includes the support module 110, the column-axis module 120, and the fixed plate module 130. This support module 110 is installed on the fixed plate module 130 so as to be able to pivot clockwise or counterclockwise with the column-axis module 120 as a pivot axis. For example, as shown in FIG. 8(a), the support module 110 may be maintained in the initial installation position parallel to the fixed plate module 130 positioned below, i.e., in a fixed state with respect to the fixed plate module 130, when the lower end of the fixing pin 134 is inserted into the fixing pin insertion hole 132 of the fixed plate module 130. Then, as shown in FIG. 8(b), when the fixing pin 134 is separated from the pivot hole 113 and the fixing pin insertion hole 132, the support module 110 may pivot clockwise around the column-axis module 120 to the pivoted position for maintenance in the direction of the left arrow. During return after maintenance, the support module 110 may pivot counterclockwise in the direction of the right arrow around the column-axis module 120 to return to the initial installation position. When the fixing pin 134 is inserted into both the pivot hole 113 and the fixing pin insertion hole 132, it can be re-fixed to the fixed plate module 130.

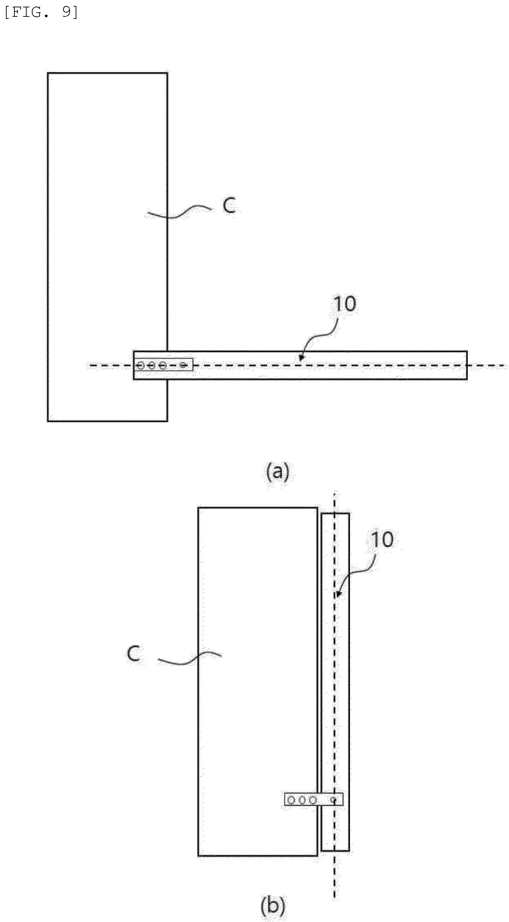

As shown in FIG. 9, the manager may easily maintain the support module 110, a plurality of pulleys 112 and 117 connected to the support module 110, and the wire detachment prevention member 115 by pivoting the support module 110 clockwise or counterclockwise. In addition, the manager may easily retrieve the wire rope 20 connected to the support module 110, and the floating sensor unit 30 connected to the wire rope 20 from the water quality enhancement tank (aeration tank) C, and replace the first sensor 331 connected to the floating sensor unit 30 with any one of the second sensor 332, the third sensor 333, or the Nth sensor 33N.

As described above, the floating module 310 includes a hat-shaped cover module 320 installed on the upper surface thereof. The hat-shaped cover module 320 prevents water and foreign substances from remaining on the cover module 320 by guiding water to flow from the upper side to the lower side when surging water touches the upper side of the cover module. The hat-shaped cover module 320 may prevent water-related dirt and foreign substances from accumulating on the cover module by allowing water and foreign substances to flow away. Through this, the manager may more easily maintain the cover module 320 and the floating module 310.

The embodiments of the present invention have been described with reference to the attached drawings. However, those skilled in the art to which the present invention pertains will understand that the invention can be implemented in other specific forms without departing from the technical spirit or essential characteristics of the present invention.

### [Description of Reference Numerals]

1: Floating water quality measuring device
10: Cantilever unit
110: Support module
111: First wire fixing ring
112: First pulley
113: Pivot hole
114: Column through-hole
115: Wire detachment prevention member
1150: Housing
1151: First wheel
1152: Second wheel
116: Second wire fixing ring
117: Second pulley
120: Column-axis module
121: Column fixing ring
130: Fixed plate module
131: Support end
132: Fixing pin insertion hole
133: Lower fixing hole
134: Fixing pin
20: Wire rope
30: Floating sensor unit
310: Floating module
320: Cover module
321: Sensor connector
330: Sensor module
40: Weight
410: First weight
420: Second weight
A: Fixed wire
B: Gap
C: Water quality enhancement tank (aeration tank)
D: Data transmission device
E: Water

## Claims

1. A floating water quality measuring device (1) comprising:
a cantilever unit (10) including a support module (110), a column-axis module (120), and a fixed plate module (130),
wherein the support module (110) includes a first wire fixing ring (111) formed on a lower surface thereof, a pivot hole (113) vertically penetrating from an upper surface to the lower surface at one end, and a column through-hole (114) vertically penetrating from the upper surface to the lower surface at a position spaced apart from the pivot hole (113) toward the other end,
wherein a lower end of the column-axis module (120) passes through the column through-hole (114) of the support module (110) and protrudes outward from the support module (110), and
wherein the fixed plate module (130) includes a support end (131) which supports the lower end of the column-axis module (120) protruding outward from the support module (110), and a fixing pin insertion hole (132) formed at a position overlapping with the pivot hole (113);
a wire rope (20) having one end connected to the first wire fixing ring (111); and
a floating sensor unit (30) including a floating module (310) configured to float on water, a cover module (320) including a sensor connector (321) formed on a lower surface thereof, which is connected at an upper surface thereof to the other end of the wire rope (20), thereby being fixed to the upper surface of the floating module (310), and a sensor module (330) having one end connected to the sensor connector (321) and the other end protruding outside the floating module (310) to measure water quality.

2. The floating water quality measuring device (1) according to claim 1, wherein the cover module (320) is formed in a hat shape inclined downward from top to bottom so that fluid flows from the upper side to the lower side.

3. The floating water quality measuring device (1) according to claim 1, wherein the support module (110) comprises:
a pulley (112) formed on a lower surface thereof; and
a wire detachment prevention member (115) including: a housing (1150) formed at a position spaced apart from the pulley (112); a first wheel (1151) installed on one side of the housing; and a second wheel (1152) installed on the other side of the housing,
wherein the wire rope (20) passes between the first wheel (1151) and the second wheel (1152).

4. The floating water quality measuring device (1) according to claim 1, wherein the column-axis module (120) comprises a column fixing ring (121) formed at an upper end thereof, to which one end of the fixing wire A is connected, and
the support module (110) comprises a second wire fixing ring (116) formed on an upper surface thereof, to which the other end of a fixing wire A connected to the column fixing ring (121) is connected.

5. The floating water quality measuring device (1) according to claim 1, wherein the sensor module (330) comprises:
a first sensor (331) configured to measure dissolved oxygen (DO) of water; and
second (332) to Nth sensor (33N) configured to measure at least one of hydrogen ion concentration (pH), oxidation-reduction potential (ORR), temperature, and chemical oxygen demand (COD) of water,
wherein the sensor module (330) is detachably coupled to the sensor connector (321).

6. The floating water quality measuring device (1) according to claim 1, wherein the fixed plate module (130) comprises at least one lower fixing hole (133) formed at a position spaced apart from the fixing pin insertion hole (132), into which a fixed bolt is inserted.

7. The floating water quality measuring device (1) according to claim 3, further comprising a first weight (410) connected to the wire rope (20) located between the first wire fixing ring (111) and the first pulley (112).
